# FASCICULE DE BREVET EUROPEEN

(11) **EP 3 906 080 B1**
(45) Date de publication et mention de la délivrance du brevet: **14.08.2024**
(21) Numéro de dépôt: 19848781.1
(22) Date de dépôt: 17.12.2019
(51) Int. Cl.: A61M 21/00

(54) **DISPOSITIF DE CONFORT VISUEL**
VORRICHTUNG FÜR VISUELLEN KOMFORT
VISUAL COMFORT DEVICE

(30) Priorité: 04.01.2019 US 201962788357 P
(43) Date de publication de la demande: 10.11.2021
(73) Titulaire: BOARDING RING, 83190 Ollioules (FR)
(72) Inventeur: JEANNIN, Hubert, 83190 OLLIOULES (FR)
(74) Mandataire: Med'inVent Consulting
(86) Numéro de dépôt international: PCT/FR2019/053131
(87) Numéro de publication internationale: WO 2020/141269

(56) Documents cités:
- DE-A1- 102017 005 982
- US-B2- 9 862 312
- US-B2- 9 994 228

## Description

### Domaine de l'invention

L'invention se rapporte à un dispositif de confort visuel. Plus particulièrement, l'invention concerne des dispositifs de repérage visuel, avantageusement latéral ou bilatéral, destinés notamment à remédier aux troubles de la vigilance, dans les situations de vide visuel ou au contraire, de confinement visuel.

### Arrière-plan technologique

Le champ visuel, du fait de la difficulté de sa stabilisation inertielle et de son faible repérage, peut provoquer de la claustrophobie, de l'agoraphobie, divers vertiges, diverses migraines, de la cinétose ou de la cyber-cinétose..., et dans de nombreux cas, de l'inconfort, du stress et ainsi, un défaut de vigilance. De tels désagréments peuvent s'avérer des inconvénients majeurs.

Préférentiellement mais non limitativement, de tels dispositifs de confort visuel peuvent être employés dans le cadre d'applications en lien avec des espaces fermés ou partiellement fermés, éventuellement mobiles, transportant par exemple des passagers y compris des animaux, tels que non limitativement des cabines, cockpit et cages de transports, des véhicules, des trains, des avions, des bateaux, mais aussi des espaces fixes ou mobiles pouvant éventuellement permettre l'affichage d'images, tels que, notamment, des salles de cinéma, des cabines d'ascenseurs et autres téléphériques, des appareils de radiologie et/ou des scanners, des simulateurs fixes ou mobiles, des masques de réalité virtuelle, mixte ou augmentée.

Une différence de perception entre la vue et l'équilibre perçu par l'oreille interne se produit lorsqu'un individu est placé dans un environnement en mouvement sans percevoir visuellement ce mouvement, et inversement. Dans une telle situation, l'œil perçoit un environnement stable à l'intérieur d'un objet en mouvement, par exemple à l'intérieur d'une cabine d'un navire en mouvement, alors que l'oreille interne perçoit l'information opposée. L'individu perçoit le mouvement du navire. Cette contradiction ou différence de perception est la cause du mal des transports comprenant non limitativement, mal de mer, mal de l'air, mal des voitures, etc., également appelé cinétose. Une telle cinétose peut également apparaître lors de l'emploi de simulateurs et/ou de masques de réalité virtuelle et/ou augmentée. En l'espèce, les informations perçues par l'oreille interne et les informations que l'individu voit sont en contradiction.

Pour pallier de tels inconvénients, différents dispositifs anti-cinétose ont été développés. Par exemple, la demande de brevet internationale WO01/22151 propose, pour lutter contre la cinétose, des lunettes contenant un dispositif d'équilibrage visuel qui est ajouté ou intégré à tout support, en l'espèce auxdites lunettes, qui doit être positionné dans le champ de vision périphérique de l'individu. Il est constitué d'un tube ou d'un tuyau ou de tout autre récipient fermé sur lui-même, imperméable, transparent ou translucide, dans lequel sont contenues au moins deux substances qui se trouvent dans des états et/ou des masses différents, tels que par exemple, l'une sous forme liquide, l'autre sous forme gazeuse, de sorte que les interfaces entre lesdites substances marquent des points de référence de niveau visibles.

Le tube fermé sur lui-même revêt la forme générale d'une bague ou d'un tore inséré ou intégré dans une lentille ou oculaire, un cadre avec ou sans lentille, voire fixé par collage ou clipsage. Au moins une des deux substances que le tube contient est un liquide, de sorte qu'elle fonctionne d'une manière fondamentalement comparable à celle de l'oreille interne.

Alternativement, le dispositif d'équilibrage visuel peut se manifester sous la forme d'une image virtuelle ou lumineuse du même type, projetée ou intégrée dans une lentille de lunettes, obtenue au moyen d'un dispositif électronique constitué par exemple par un capteur, sous la forme d'un gyroscope ou de toute autre source capteur d'information d'une position environnementale ou mobile, agencé ou adapté pour détecter les variations de la position par rapport à la gravité. Les informations, éventuellement après traitement par un ordinateur, sont ensuite mises à la disposition de l'œil ou des yeux d'un utilisateur par un système d'imagerie ou d'éclairage, par exemple, sous la forme d'un périmètre intérieur ou extérieur d'un écran, ou sous la forme d'une animation ou encore par un ou plusieurs écrans muraux ou non, voire sous forme de système d'éclairage, par exemple par faisceaux lumineux, voire sous forme de colonnes ou d'affichages à l'aide de diodes électroluminescentes.

Dans de tels dispositifs, des problèmes de perception pourraient survenir, du fait, par exemple, des variations du niveau d'éclairage. Par conséquent, les dispositifs peuvent être moins efficace en raison d'un mauvais agencement périphérique du contraste ou de l'éclairage. En outre, les informations délivrées par de tels dispositifs anti-cinétose s'avèrent trop nombreuses et ainsi très difficiles à discriminer, lors de leur analyse, pour pouvoir obtenir l'efficacité escomptée.

Lorsqu'il est rapporté dans le champ visuel, comme naturellement dans un quelconque paysage, de multiples informations visuelles, notamment une quantité importante de messages inertiels, la stratégie de perception mise en place intuitivement est moins immédiate, très complexe, et difficile d'accès. En effet, plus la quantité d'informations est importante, plus elle est encombrante, rendant alors le repérage plus difficile et ainsi ralenti, le volume d'informations inertielles brouillant, par son abondance, son efficacité.

Le brevet US9,862,312 divulgue une autre solution pour résoudre le mal des transports en utilisant des indicateurs visuels pour simuler les mouvements d'un véhicule.

### Résumé de l'invention

L'invention, de par sa structure et sa composition, permet de résoudre tout ou partie des inconvénients précédemment mentionnés.

Plus particulièrement, un dispositif de confort visuel conforme à l'invention remédie à de telles difficultés, en proposant un nouvel agencement et une nouvelle organisation d'une information visuelle réduite à son plus simple minimum, rapidement accessible et analysable par le cerveau d'un utilisateur humain ou animal. L'information visuelle mise à disposition, par un dispositif de confort visuel conforme à l'invention, sous la forme d'un contenu ergonomique, simplifié et résumé, induit en conséquence un confort et une vigilance qui vont bien au-delà de la réduction, voire même de la suppression de la cinétose proposées par les dispositifs actuels.

Préférentiellement mais non limitativement, un dispositif de confort visuel conforme à l'invention peut être employé dans le cadre d'application en lien avec des espaces fermés ou partiellement fermés, éventuellement mobiles, transportant par exemple des passagers y compris des animaux, tels que non limitativement des cabines, cockpit et cages de transports, des véhicules, des trains, des avions, des bateaux, mais aussi des espaces fixes ou mobiles pouvant éventuellement permettre l'affichage d'images, tels que, notamment, des appareils de radiologie et/ou des scanners, des salles de cinéma, des cabines d'ascenseurs et autres téléphériques, des simulateurs fixes ou mobiles, des masques de réalité virtuelle, mixte ou augmentée.

Plus particulièrement, l'invention propose un dispositif de confort visuel comportant au moins un capteur inertiel, une unité de traitement et au moins un écran. L'écran est destiné à être positionné latéralement dans un champ de vision périphérique d'un utilisateur. L'unité de traitement est couplée au capteur inertiel et à l'écran. Le dispositif est configuré pour afficher sur l'écran une grille inertielle représentative d'une information inertielle, ladite grille inertielle affichée comportant un maximum de seize points et/ou croisements de ligne.

Selon un mode de réalisation préféré, le dispositif peut comporter deux écrans agencés pour être placés dans le champ monoculaire extérieur de chaque œil d'un utilisateur, chaque écran affichant une grille inertielle représentative de l'information inertielle.

Afin d'être plus efficace, la grille inertielle affichée sur un écran peut comporter au plus neuf points et/ou croisements de lignes.

Selon un mode de réalisation particulier, le capteur inertiel peut être fixé solidairement à l'écran et peut fournir l'information inertielle à l'unité de traitement, l'information inertielle étant représentative d'une orientation inertielle dudit écran.

Le capteur inertiel peut comprendre un ou plusieurs des types de capteurs choisis dans la liste suivante : un ou plusieurs accéléromètres, gyroscope, centrale inertielle, détecteur de champ magnétique.

L'unité de traitement peut être configurée pour produire une image comportant la grille inertielle orientée et déplacée sur l'écran en fonction de l'information inertielle reçue du capteur.

Plus particulièrement, l'unité de traitement peut être configurée pour :
- produire une grille virtuelle autour de l'utilisateur ayant une position fixe dans l'espace,
- positionner l'écran par rapport à la grille virtuelle en fonction de l'information inertielle, et
- extraire l'image représentative de la grille inertielle à afficher sur l'écran en fonction du positionnement de l'écran.

Afin de mettre en œuvre le dispositif dans un véhicule, le dispositif peut comporter en outre un moyen de fixation pour siège de véhicule et au moins un bras de liaison reliant l'au moins un écran au moyen de fixation de sorte que l'écran soit placé au niveau d'un côté de la tête d'un utilisateur du siège afin que l'écran soit placé dans le champ de vision périphérique dudit utilisateur

Selon un exemple de réalisation, l'invention peut être une paire de lunettes comprenant deux branches destinées à coopérer avec des oreilles d'un utilisateur, ladite paire de lunette comportant au moins un dispositif de confort visuel, au moins un écran étant fixé sur une des branches de sorte que l'écran soit placé latéralement dans le champ de vision périphérique de l'utilisateur.

Selon un autre exemple de réalisation, l'invention peut être un masque de réalité virtuelle comprenant un boîtier destiné à être placé dans le champ de vison d'un utilisateur et comportant au moins un écran principal placé dans le champ de vision central d'un utilisateur, ledit masque comportant un dispositif de confort visuel dans lequel les deux écrans peuvent être placés latéralement à l'intérieur du boîtier de sorte que ceux-ci soient placés dans le champ de vision périphérique respectivement d'un oeil droit et d'un oeil gauche de l'utilisateur.

### Brève description des figures

L'invention sera mieux comprise et d'autres caractéristiques et avantages de celle-ci apparaîtront à la lecture de la description suivante de modes de réalisation particuliers de l'invention, donnés à titre d'exemples illustratifs et non limitatifs, et faisant référence aux dessins annexés, parmi lesquels :
la figure 1 représente un premier mode de réalisation de l'invention sous forme de lunettes,
la figure 2 représente un deuxième mode de réalisation de l'invention sous forme de masque de réalité virtuelle,
la figure 3 représente un troisième mode de réalisation de l'invention destiné à être fixer à un siège de véhicule,
la figure 4 représente un schéma fonctionnel de l'invention,
les figures 5 à 7 illustrent un premier mode de réalisation préférée d'une grille inertielle conforme à l'invention,
les figures 8 et 9 illustrent un deuxième mode de réalisation préférée d'une grille inertielle conforme à l'invention,
les figures 10 et 11 illustrent un troisième mode de réalisation préférée d'une grille inertielle conforme à l'invention.

### Description détaillée

Sur la considération que le champ visuel périphérique est plus particulièrement dédié à cette fonction de grille de lecture, ou de support visuel inertiel permettant tout à la fois, la structuration, la localisation et la poursuite de la focalisation, un dispositif de confort visuel conforme à l'invention apporte, par la voie visuelle monoculaire, latérale, mais sagittale, un système d'informations caractérisé par des stimulations visuelles les plus simplifiées possible et ainsi les plus aisément accessibles à une franche perception.

En effet, un dispositif de confort visuel conforme à l'invention est notamment caractérisé par la grande simplicité du message visuel qu'il propose, plus particulièrement du fait de son agencement et sa mise à disposition. De par l'agencement et la composition avantageux d'un tel dispositif de confort visuel, ce dernier permet alors la mise à disposition d'une information visuelle claire et concise, par conséquent une perception par le cerveau d'un utilisateur humain ou animal plus simple et plus rapide de ladite information, ainsi qu'une plus simple et rapide analyse par le cerveau d'un utilisateur humain ou animal, c'est-à-dire une meilleure intégration perceptive et une meilleure efficacité.

Un dispositif de confort visuel conforme à l'invention peut être agencé de sorte à ce qu'il produise et affiche une information inertielle, sous la forme d'une grille inertielle, apportée par un maximum de seize points ou de croisements de lignes, par côté ou œil, ou encore par un maximum de trois lignes de longueurs variables, par côté ou œil. Préférentiellement, afin d'apporter seulement l'information nécessaire à chaque œil, une telle information inertielle peut être agencée pour être apportée sous la forme de neuf points. De telles images ainsi résumées, permettent alors une perception plus rapide de l'information inertielle apportée dans le champ visuel.

Selon un exemple préféré mais non limitatif d'un dispositif de confort visuel conforme à l'invention, ce dernier peut comporter un ou plusieurs écrans latéraux et sagittaux, c'est-à-dire orientés d'avant en arrière ou d'arrière en avant, rapportés très latéralement, agencés de sorte à être employés conjointement ou successivement pour chaque œil et disposés dans le champ visuel monoculaire extérieur de chaque œil, tels des œillères de chevaux, pour apporter à un ou deux yeux, une information visuelle affichée sous la forme de points ou de lignes, de manière résumée et réduite. Lesdits écrans peuvent ainsi recouvrir une partie du champ visuel monoculaire de chaque œil. Éventuellement, en variante ou en complément, pour améliorer la vigilance et la perception du monde extérieur d'un utilisateur, que celui-ci soit non limitativement un pilote, un chauffeur et/ou tout autre opérateur embarqué, d'un dispositif de confort visuel conforme à l'invention, le ou les écrans de ce dernier peuvent être avantageusement translucides, voire même transparents.

Préférentiellement mais non limitativement, pour maintenir lesdits écrans dans une position déterminée et optimale, c'est-à-dire dans le champ visuel monoculaire extérieur de chaque œil, un dispositif de confort visuel conforme à l'invention peut comprendre un support coopérant solidairement, selon toute liaison mécanique adaptée, telle que, par exemple, une liaison encastrement, pivot, rotule ou encore glissière, avec le ou les écrans et agencés de sorte à maintenir lesdits écrans dans une position déterminée, telle que mentionnés précédemment. Éventuellement, afin de faciliter la fabrication d'un dispositif de confort visuel conforme à l'invention, le support de ce dernier peut comprendre, de manière avantageuse mais non limitative, un bandeau, un bonnet, un chapeau, un casque, un masque ou encore une structure similaire ou identique à la forme générale d'une monture de lunettes avec ou sans oculaire(s).

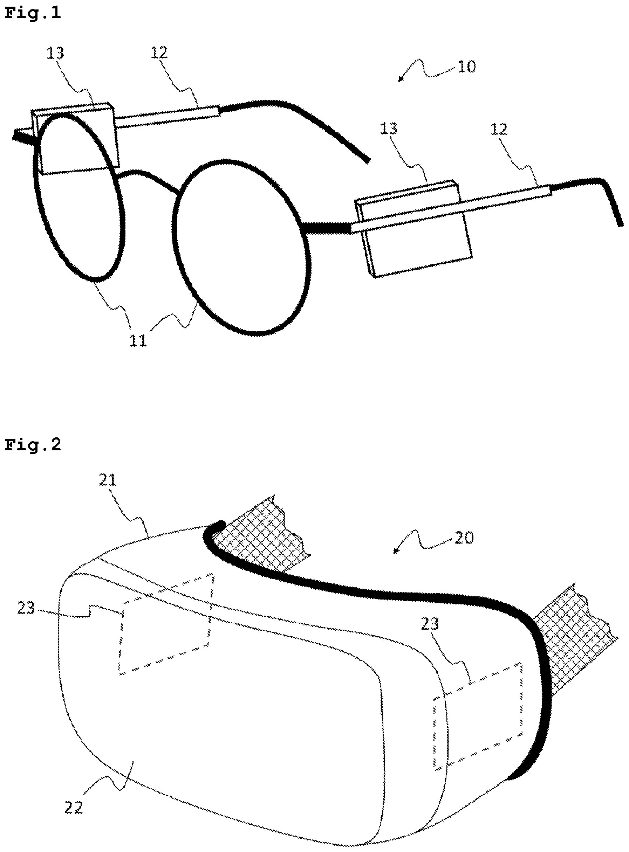

La figure 1 montre un exemple de dispositif de confort visuel sous la forme d'une paire de lunettes 10 conforme à l'invention qui comporte deux oculaires 11 reliés à deux branches 12 destinées à coopérer avec les oreilles d'un utilisateur afin de maintenir la paire de lunette dans une position où les oculaires 11 se trouvent placés dans le champ de vision central de l'utilisateur. Deux écrans 13 sont placés sur les branches 12 des lunettes afin que ceux-ci soient placés dans le champ de vision périphérique de l'utilisateur de ladite paire de lunettes 10.

La figure 2 montre un exemple de dispositif de confort visuel sous la forme d'un masque de réalité virtuelle 20 comprenant un boîtier 21 destiné à être placé dans le champ de vison d'un utilisateur et comportant au moins un écran principal 22 placé dans le champ de vision central d'un utilisateur. Deux écrans 23 sont placés latéralement à l'intérieur du boîtier 21 de sorte que ceux-ci soient placés dans le champ de vision périphérique respectivement de l'œil droit et de l'œil gauche de l'utilisateur.

Selon différents modes de réalisation, l'invention prévoit que les écrans 13 et 23 précédemment mentionnés d'un dispositif de confort visuel 10 ou 20 conforme à l'invention puissent coopérer solidairement, selon toute liaison mécanique adaptée, telle que, par exemple, une liaison encastrement, pivot, rotule ou encore glissière, avec le ou les écrans et agencés de sorte à maintenir lesdits écrans 13 et 23 dans une position déterminée relativement aux types de support, tel un masque 20, une paire de lunettes 10 ou un simulateur de réalité virtuelle, mixte ou augmentée, voire même être directement intégré audit support.

En variante, selon le cadre d'application ou en fonction des besoins, notamment en lien avec des espaces confinés créés par un support fixe ou mobile, tels que par exemples des cabines d'avions ou plus généralement de véhicules, ou encore, éventuellement un peu plus éloignés de la tête, de tels écrans peuvent coopérer solidairement avec un support, selon toute liaison mécanique adaptée, telle que, par exemple, une liaison encastrement, pivot, rotule ou encore glissière, et agencés de sorte à maintenir lesdits écrans dans une position déterminée, avec tout ou partie du support de sorte à être maintenus dans une position déterminée. A titre d'exemples non limitatifs, lorsqu'un dispositif de confort visuel conforme à l'invention est employé en lien avec la cabine d'un cockpit ou plus généralement d'un véhicule, lesdits écrans peuvent être alors maintenus à partir du plafond ou encore coopérer avec le siège d'un utilisateur, par exemple l'appui-tête d'un passager ou conducteur ou plus généralement d'un utilisateur dudit dispositif, ou encore un siège auto pour enfant lorsque l'utilisateur dudit dispositif est un enfant. Pour ce faire, un dispositif de confort visuel conforme à l'invention peut être agencé tel des œillères coopérant avec le siège, par exemple l'appui-tête, d'un passager. Également, en variante ou en complément, pour s'adapter en fonction d'un passager ou plus généralement d'un utilisateur, que celui-ci nécessite ou non de manière permanente ou temporaire l'emploi dudit dispositif de confort visuel, l'invention prévoit qu'un tel dispositif, plus particulièrement les écrans, peuvent être, par tout moyen, escamotables, rétractables ou amovibles.

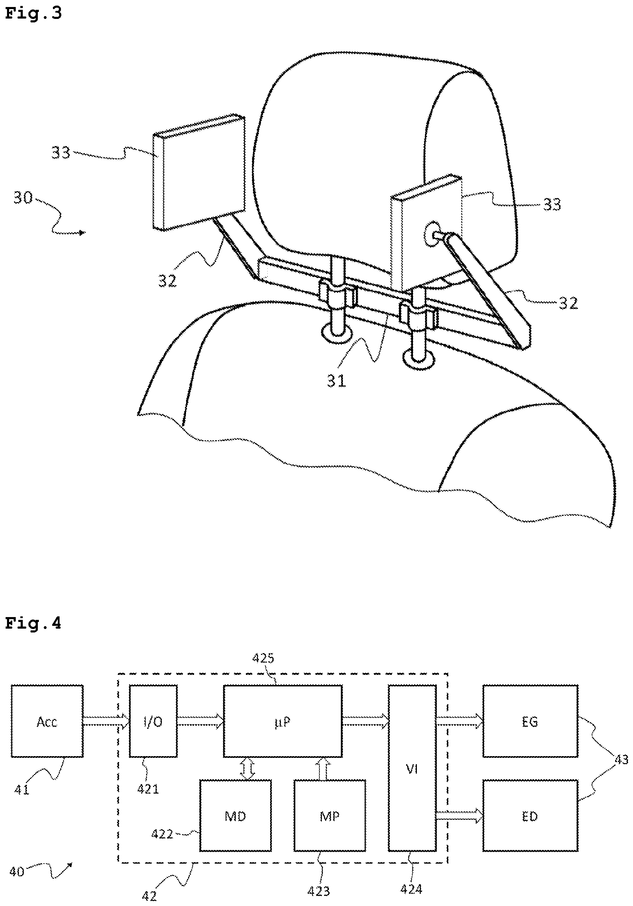

La figure 3 montre un exemple de dispositif de confort visuel 30 pour un siège de véhicule. Le dispositif 30 comporte un moyen de fixation 31 permettant de rendre le dispositif 30 solidaire d'un siège d'un véhicule et deux bras de liaison 32 reliant respectivement deux écrans 23 au moyen de fixation 31. Le moyen de fixation 31 est par exemple constitué d'une barre munie de deux colliers de serrage venant coopérer avec les montants de fixation d'un appui-tête. Les bras de liaison peuvent être des bras mobiles par rapport au moyen de fixation 31 et aux écrans 33 de sorte que les écrans puissent être ajusté afin d'être placé au niveau d'un côté de la tête d'un utilisateur du siège dans le champ de vision périphérique dudit utilisateur. A titre d'exemple non limitatif, la liaison entre le moyen de fixation 31 et chacun des bras de liaison 32 peut être une liaison pivot alors que la liaison entre un bras 32 et un écran 33 peut être une liaison rotule.

Un dispositif de confort visuel conforme à l'invention peut comporter un ensemble d'un ou deux écrans rapportés sur le côté extérieur des oculaires dudit masque, desdites lunettes ou dudit simulateur, diffusant tout ou partie des informations minimalistes et rapidement perceptibles par le cerveau humain, et éventuellement d'éléments électroniques, comme par exemple un ou plusieurs capteurs inertiels et/ou magnétique(s), une source d'énergie éventuelle, et une unité de traitement, de tels éléments coopérant ensemble par couplage.

La figure 4 détaille un schéma fonctionnel d'un dispositif de confort visuel 40 tel qu'il pourrait être mis en oeuvre dans la paire de lunettes 10, le masque 20 ou le dispositif 30 précédemment décrit. Le dispositif 40 comporte principalement un capteur inertiel 41 connecté à une unité de traitement 42 elle-même connectée à deux écrans 43.

Le capteur inertiel 41 sert à mesurer une information inertielle subie par le dispositif de confort visuel 40. Cette information inertielle peut correspondre à l'information inertielle ressentie par l'utilisateur lorsque le dispositif de confort visuel est solidaire de l'utilisateur, tel que par exemple une paire de lunette ou un masque ou correspondre à une information inertielle de l'environnement de l'utilisateur lorsque le dispositif est fixé à l'environnement de l'utilisateur. Le capteur inertiel 41 peut être de différents types tel que par exemple une centrale inertielle, un gyroscope, un ou plusieurs accéléromètres, ou encore un détecteur de champ magnétique terrestre ou d'une combinaison de ces différents types de capteurs. L'important est que le capteur inertiel 41 puisse fournir une information représentative d'un mouvement subi et/ou d'une accélération subie par le dispositif afin de pouvoir élaborer une grille inertielle représentative de ce mouvement.

L'unité de traitement 42 comporte une interface d'entrée/sortie 421, une mémoire de données 422, une mémoire de programmes 423 et une interface vidéo 424 reliées à un microprocesseur 425. Selon un mode de réalisation préféré, l'unité de traitement 42 est un circuit de type microcontrôleur qui intègre les circuits 421 à 425 ou des circuits équivalents pour réaliser la même fonction. L'interface d'entrée/sortie 421 est connectée au capteur inertiel 41 par une liaison filaire ou sans fil d'un type connu afin de recevoir du capteur inertiel 41 et de fournir au microprocesseur des données inertielles échantillonnées. La mémoire de données 422 est une mémoire de travail, par exemple de type RAM, qui permet de mémoriser toutes les données utilisées et calculées par le microprocesseur 425. La mémoire de programmes 423 est une mémoire non volatile qui mémorise les programmes mis en œuvre par le microprocesseur 425 ainsi que des données de configuration qui permettent d'initialiser la mémoire de données lors de la mise en activité du dispositif de confort visuel. L'interface vidéo 424 reçoit des données du microprocesseur 425 qui correspondent à des images à afficher sur les écrans 43. L'interface vidéo 424 met en forme des signaux de commande et d'image afin de les fournir auxdits écrans 43.

Les écrans 43 peuvent être de très petite dimension, par exemple de l'ordre du pouce en diagonale, si l'on souhaite les intégrer dans une paire de lunette ou de dimension un peu plus importante, par exemple de l'ordre de cinq pouces en diagonale, si l'on souhaite les fixer à un appui-tête. Une résolution de 320x200 pixels est suffisante pour afficher une grille inertielle de référence. Néanmoins, une résolution inférieure peut être utilisée si la grille ne comporte que des points et une résolution supérieure être utilisée pour avoir une meilleure fluidité de mouvement pour une grille comportant des croisements de lignes.

Parmi les programmes contenus dans la mémoire de programmes 423, un programme qui comprend une pluralité d'instructions dont l'exécution par le microprocesseur 425 met en œuvre un procédé de transformation de l'information inertielle fournie par le capteur inertiel 41 en au moins une image à afficher sur un écran 43.

Selon un premier mode de réalisation, le procédé mis en œuvre par le microprocesseur 425 initialise dans la mémoire de donnée 422 une grille inertielle à afficher sur l'écran. Puis, à réception d'une information inertielle, le microprocesseur 425 calcule un déplacement de la grille inertielle sur l'écran 43 correspondant au mouvement inverse de celui indiqué par l'information inertielle.

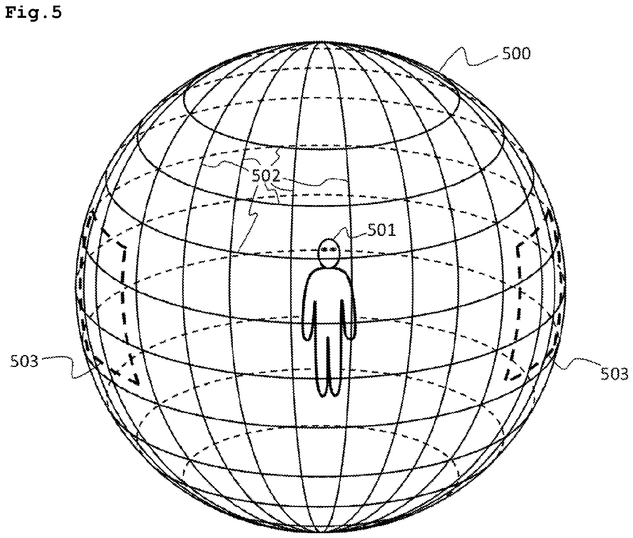

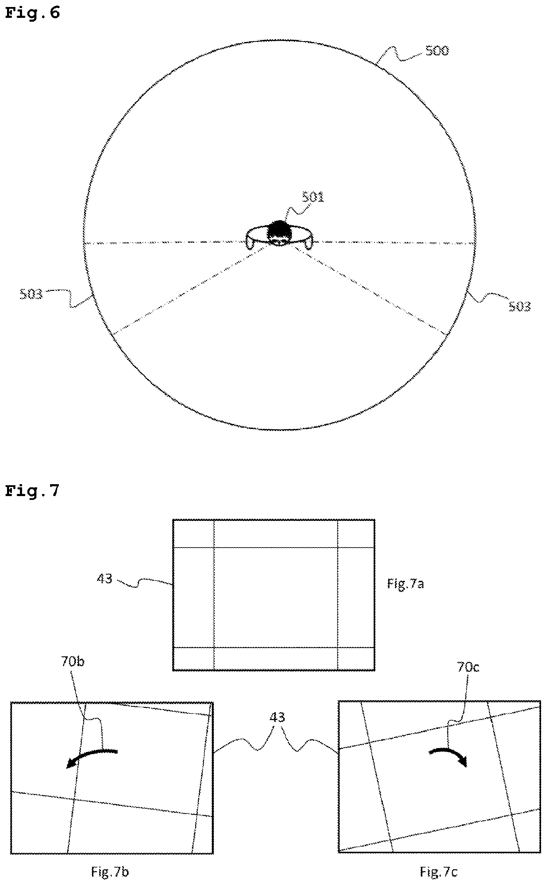

Les figures 5 à 7 illustre un deuxième mode de réalisation dans lequel le dispositif de confort visuel 40 est solidaire de l'utilisateur tel que par exemple dans le cas de la paire de lunette 10 ou le masque de réalité virtuelle 20. Le procédé mis en œuvre par le microprocesseur 425 peut consister en la création d'une constellation ou grille virtuelle 500 autour de l'utilisateur 501, ladite grille virtuelle 500 ayant une position fixe dans l'espace entourant l'utilisateur 501. Dans l'exemple de la figure 5, la grille virtuelle 500 correspond à une sphère comportant une pluralité de lignes 502 entrecroisées de sorte à fournir à l'utilisateur 501 une grille inertielle formée de croisements de lignes. Le microprocesseur 425 détermine ensuite une position de l'utilisateur 501 à l'intérieure de la constellation virtuelle 500 en fonction de l'information inertielle. La position de l'utilisateur 501 étant déterminée, des fenêtres 503 peuvent être positionnées sur la grille virtuelle 500 pour correspondre au champ de vision périphérique de l'utilisateur 501 dans lequel sont placés les écrans 43, comme montré sur la figure 6. Le microprocesseur 425 extrait ensuite de la grille virtuelle 500 les images correspondant aux fenêtres 503 pour les fournir à l'interface vidéo 424 afin de les afficher sur les écrans 43.

La figure 7 illustre des images affichées sur un écran 43 en fonction d'informations inertielle. La figure 7a montre une grille inertielle correspondant par exemple à une position d'initialisation. La position d'initialisation peut correspondre au positionnement de la grille par rapport à l'utilisateur lors de la mise sous tension du dispositif de confort visuel 40 ou lors d'un appui sur un bouton poussoir non représenté présent sur le dispositif afin de permettre son initialisation. Le microprocesseur 425 enregistre alors l'information inertielle fournie par le capteur inertiel 41 comme étant la position fixe de la grille virtuelle 500. L'image ainsi obtenue correspond à une position de l'utilisateur 501 qui est placé comme montré sur la figure 5.

Après l'initialisation du dispositif, l'information inertielle est comparée à l'information inertielle d'initialisation pour indiquer un déplacement de l'utilisateur 501 par rapport à ladite position d'initialisation, tel que par exemple illustré par les flèches 70b et 70c respectivement sur les figures 7b et 7c. Le microprocesseur 425 calcule la position de l'utilisateur 501 par rapport à la grille virtuelle en fonction du déplacement ainsi déterminé. Des images correspondant au déplacement peuvent ensuite être extraites et affichées sur l'écran 43 comme montré respectivement sur les figures 7b et 7c.

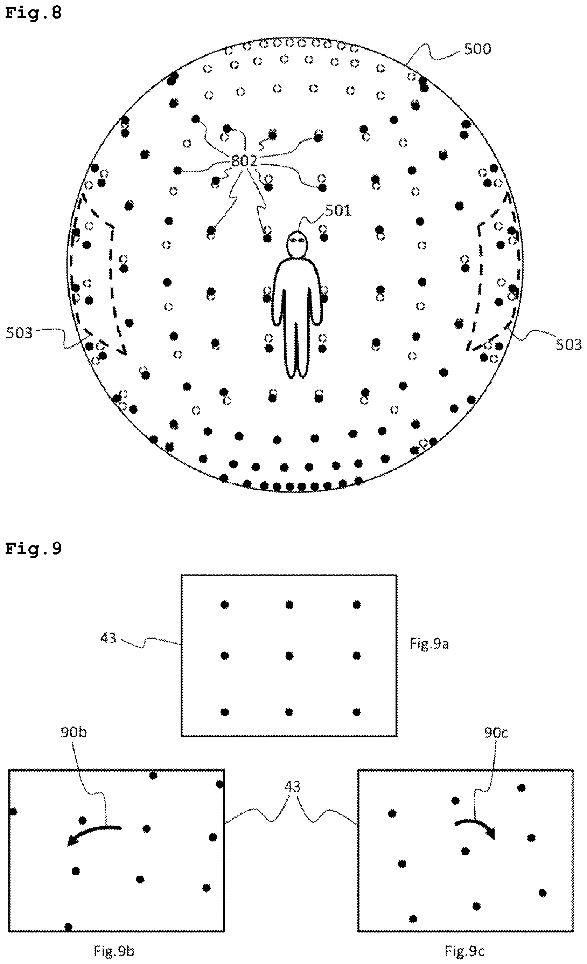

Ce mode de réalisation peut être adapté à différents autres types de grille inertielle. Les figures 8 et 9 illustrent un procédé similaire pour obtenir une grille inertielle constituée de points. La grille virtuelle 500 de la figure 8 est constituée d'une multitude de points 802. Le positionnement des fenêtres 503 se fait de la même manière que précédemment décrit en lien avec les figures 5 à 7. Le microprocesseur 425 peut ensuite extraire et afficher sur les écrans 43 les images représentatives de la grille inertielle représentées sur la figure 9. La figure 9a correspond à la position d'initialisation et les figures 9b et 9c correspondent aux images de la grille inertielle affectées d'un mouvement représenté par les flèches 90b et 90c.

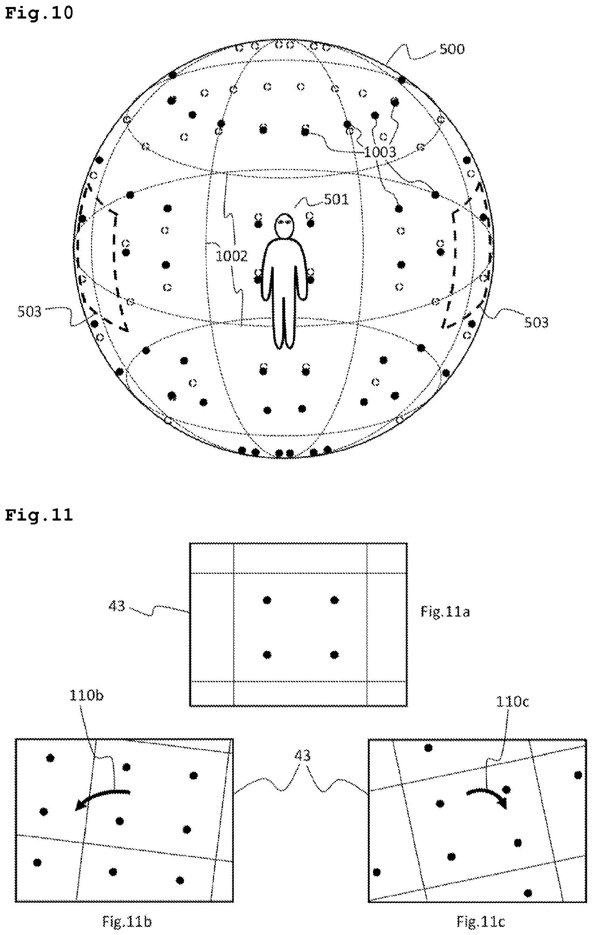

Selon une variante, les figures 10 et 11 illustrent un procédé similaire pour obtenir une grille inertielle constituée d'un mélange de croisements de lignes et de points. La grille virtuelle 500 de la figure 10 est constituée d'une pluralité de lignes 1002 et d'une multitude de points 1003. Le positionnement des fenêtres 503 se fait de la même manière que précédemment décrit en lien avec les figures 5 à 7. Le microprocesseur 425 peut ensuite extraire et afficher sur les écrans 43 les images représentatives de la grille inertielle représentées sur la figure 11. La figure 11a correspond à la position d'initialisation et les figures 11b et 11c correspondent aux images de la grille inertielle affectées d'un mouvement représenté par les flèches 110b et 110c.

Toute autre forme de grille inertielle reste possible dès lors que le nombre de points et/ou de croisements de lignes affichés sur un écran reste limité afin de ne pas fournir trop d'information dans le champ de vision périphérique d'un utilisateur.

Les exemples de mise en œuvre détaillés à l'aide des figure 5 à 11 font référence à un dispositif de confort visuel solidaire de l'utilisateur. Ainsi, tout mouvement du dispositif correspond à un mouvement de l'utilisateur. Le mouvement de la grille inertielle affichée sur les écrans 43, quant à lui, correspond à un mouvement ressenti par l'utilisateur. Alternativement, lorsque le dispositif de confort visuel 40 n'est pas solidaire de l'utilisateur, par exemple lorsqu'il est fixé à un siège, l'affichage de la grille inertielle doit correspondre à un mouvement ressenti par le siège. En effet, le mouvement ressenti par le siège est le même que le mouvement ressenti par l'utilisateur si l'utilisateur est immobile par rapport au siège. Lorsque l'utilisateur bouge par rapport au siège, la grille inertielle affichée est représentative du mouvement du siège et constitue une référence visuelle qui s'ajoute au mouvement de l'utilisateur par rapport au siège. L'important est que l'image de la grille inertielle soit représentative du mouvement ressenti par l'écran 13, 23, 33 ou 43 qui l'affiche de sorte que l'utilisateur puisse avoir une correspondance avec le mouvement ressenti par son oreille interne.

Le dispositif de confort visuel 40 a été décrit avec deux écrans. Toutefois, un seul écran peut suffire car il est possible d'intégrer le capteur inertiel 41 et l'unité de traitement 42 dans le boîtier d'un écran 43. Ainsi un dispositif utilisant deux écrans peut comporter deux dispositifs 40 indépendants l'un de l'autre mais réalisant le même type de traitement.

L'unité de traitement 42 peut également être remplacée par tout autre unité de traitement dès lors que celle-ci est en mesure de transformer une information de mesure inertielle en une image en grille inertielle.

## Revendications

1. Dispositif de confort visuel (10, 20, 30, 40) comportant au moins un capteur inertiel (41), une unité de traitement (42) et au moins un écran (13, 23, 33, 43), l'écran (13, 23, 33, 43) étant destiné à être positionné latéralement dans un champ de vision périphérique d'un utilisateur, l'unité de traitement (42) étant couplée au capteur inertiel (41) et à l'écran (13, 23, 33, 43), **caractérisé en ce que** le dispositif (10, 20, 30, 40) est configuré pour afficher sur l'écran (13, 23, 33, 43) une grille inertielle représentative d'une information inertielle, ladite grille inertielle affichée comportant un maximum de seize points et/ou croisements de lignes.

2. Dispositif selon la revendication 1, comportant deux écrans (13, 23, 33, 43) agencés pour être placés dans le champ monoculaire extérieur de chaque œil d'un utilisateur, chaque écran affichant une grille inertielle représentative de l'information inertielle.

3. Dispositif selon la revendication précédente, dans lequel la grille inertielle affichée sur un écran (43) comporte au plus neuf points et/ou croisements de lignes.

4. Dispositif selon l'une des revendications précédentes dans lequel le capteur inertiel (41) est fixé solidairement à l'écran (13, 23, 33, 43) et fournit l'information inertielle à l'unité de traitement (42), l'information inertielle étant représentative d'une orientation inertielle dudit écran (13, 23, 33, 43) .

5. Dispositif selon l'une des revendications précédentes, dans lequel le capteur inertiel (41) comprend un ou plusieurs des types de capteurs choisis dans la liste suivante :
- un ou plusieurs accéléromètres,
- gyroscope,
- centrale inertielle,
- détecteur de champ magnétique.

6. Dispositif selon l'une des revendications 4 ou 5, dans lequel l'unité de traitement (42) est configurée pour produire une image comportant la grille inertielle orientée et déplacée sur l'écran (13, 23, 33, 43) en fonction de l'information inertielle reçue du capteur (41).

7. Dispositif selon la revendication précédente, dans lequel l'unité de traitement (42) est configurée pour :
- produire une grille virtuelle (500) autour de l'utilisateur ayant une position fixe dans l'espace,
- positionner l'écran par rapport à la grille virtuelle en fonction de l'information inertielle, et
- extraire l'image représentative de la grille inertielle à afficher sur l'écran en fonction du positionnement de l'écran.

8. Dispositif selon l'une des revendications précédentes, comportant en outre un moyen de fixation (31) pour siège de véhicule et au moins un bras de liaison (32) reliant l'au moins un écran (33) au moyen de fixation (31) de sorte que l'écran (33) soit placé au niveau d'un côté de la tête d'un utilisateur du siège afin que l'écran (33) soit placé dans le champ de vision périphérique dudit utilisateur.

9. Paire de lunettes (10) comprenant deux branches (12) destinées à coopérer avec des oreilles d'un utilisateur **caractérisé en ce qu'**elle comporte au moins un dispositif selon l'une des revendications 1 à 7, au moins un écran (13) étant fixé sur une des branches (12) de sorte que l'écran soit placé latéralement dans le champ de vision périphérique de l'utilisateur.

10. Masque de réalité virtuelle (20) comprenant un boîtier (21) destiné à être placé dans le champ de vison d'un utilisateur et comportant au moins un écran principal (22) placé dans le champ de vision central d'un utilisateur **caractérisé en ce qu'**il comporte un dispositif selon l'une des revendications 2 à 7 et dans lequel les deux écrans (23) sont placés latéralement à l'intérieur du boîtier de sorte que ceux-ci soient placés dans le champ de vision périphérique respectivement d'un œil droit et d'un œil gauche de l'utilisateur.

## Patentansprüche

1. Vorrichtung (10, 20, 30, 40) für visuellen Komfort, die mindestens einen Trägheitssensor (41), eine Verarbeitungseinheit (42) und mindestens einen Bildschirm (13, 23, 33, 43) umfasst, wobei der Bildschirm (13, 23, 33, 43) zum lateralen Positionieren in einem peripheren Sichtfeld eines Benutzers bestimmt ist, wobei die Verarbeitungseinheit (42) mit dem Trägheitssensor (41) und dem Bildschirm (13, 23, 33, 43) gekoppelt ist, **dadurch gekennzeichnet, dass** die Vorrichtung (10, 20, 30, 40) zum Anzeigen eines Trägheitsgitters auf dem Bildschirm (13, 23, 33, 43) konfiguriert ist, das für eine Trägheitsinformation repräsentativ ist, wobei das genannte angezeigte Trägheitsgitter maximal sechzehn Punkte und/oder Linienkreuzungen umfasst.

2. Vorrichtung nach Anspruch 1, die zwei Bildschirme (13, 23, 33, 43) umfasst, die zum Platzieren im äußeren monokularen Feld jedes Auges eines Benutzers ausgelegt sind, wobei jeder Bildschirm ein für die Trägheitsinformation repräsentatives Trägheitsgitter anzeigt.

3. Vorrichtung nach dem vorherigen Anspruch, bei der das auf einem Bildschirm (43) angezeigte Trägheitsgitter höchstens neun Punkte und/oder Linienkreuzungen umfasst.

4. Vorrichtung nach einem der vorherigen Ansprüche, bei der der Trägheitssensor (41) fest mit dem Bildschirm (13, 23, 33, 43) verbunden ist und die Trägheitsinformation an die Verarbeitungseinheit (42) liefert, wobei die Trägheitsinformation für eine Trägheitsorientierung des genannten Bildschirms (13, 23, 33, 43) repräsentativ ist.

5. Vorrichtung nach einem der vorherigen Ansprüche, bei der der Trägheitssensor (41) einen oder mehrere der Sensortypen umfasst, die aus der folgenden Liste ausgewählt sind:
- ein oder mehrere Beschleunigungssensoren,
- Gyroskop,
- Trägheitszentrale,
- Magnetfelddetektor.

6. Vorrichtung nach einem der Ansprüche 4 oder 5, bei der die Verarbeitungseinheit (42) zum Erzeugen eines Bildes konfiguriert ist, das das Trägheitsgitter auf dem Bildschirm (13, 23, 33, 43) in Abhängigkeit von der vom Sensor (41) empfangenen Trägheitsinformation orientiert und verschoben enthält.

7. Vorrichtung nach dem vorherigen Anspruch, bei der die Verarbeitungseinheit (42) konfiguriert ist zum:
- Erzeugen eines virtuellen Gitters (500) um den Benutzer herum mit einer festen Position im Raum,
- Positionieren des Bildschirms in Bezug auf das virtuelle Gitter in Abhängigkeit von der Trägheitsinformation, und
- Extrahieren des für das auf dem Bildschirm anzuzeigende Trägheitsgitter repräsentativen Bildes in Abhängigkeit von der Positionierung des Bildschirms.

8. Vorrichtung nach einem der vorherigen Ansprüche, die außerdem ein Befestigungsmittel (31) für einen Fahrzeugsitz und mindestens einen Verbindungsarm (32) umfasst, der den mindestens einen Bildschirm (33) mit dem Befestigungsmittel (31) verbindet, so dass der Bildschirm (33) auf der Höhe einer Seite des Kopfs eines Benutzers des Sitzes platziert ist, damit der Bildschirm (33) im peripheren Sichtfeld des genannten Benutzers platziert ist.

9. Brille (10) mit zwei Bügeln (12), die zum Zusammenwirken mit den Ohren eines Benutzers bestimmt sind, **dadurch gekennzeichnet, dass** sie mindestens eine Vorrichtung nach einem der Ansprüche 1 bis 7 umfasst, wobei mindestens ein Bildschirm (13) an einem der Bügel (12) befestigt ist, so dass der Bildschirm lateral im peripheren Sichtfeld des Benutzers platziert ist.

10. Virtuelle Realitätsmaske (20) mit einem Gehäuse (21), das zum Platzieren im Sichtfeld eines Benutzers bestimmt ist und mindestens einen im zentralen Sichtfeld eines Benutzers platzierten Hauptbildschirm (22) umfasst, **dadurch gekennzeichnet, dass** sie eine Vorrichtung nach einem der Ansprüche 2 bis 7 umfasst, bei der die beiden Bildschirme (23) lateral im Inneren des Gehäuses platziert sind, so dass diese im peripheren Sichtfeld eines rechten Auges bzw. eines linken Auges des Benutzers platziert sind.

## Claims

1. Visual comfort device (10, 20, 30, 40) containing at least one inertial sensor (41), a processing unit (42) and at least one screen (13, 23, 33, 43), the screen (13, 23, 33, 43) being intended to be positioned laterally in a peripheral visual field of a user, the processing unit (42) being coupled to the inertial sensor (41) and to the screen (13, 23, 33, 43), **characterized in that** the device (10, 20, 30, 40) is configured to display on the screen (13, 23, 33, 43) an inertial matrix representative of an item of inertial information, said displayed inertial matrix containing a maximum of sixteen points and/or line intersections.

2. Device according to claim 1, containing two screens (13, 23, 33, 43) arranged to be placed in the outer monocular field of each eye of a user, each screen displaying an inertial matrix representing the inertial information.

3. Device according to the preceding claim, in which the inertial matrix displayed on a screen (43) contains at most nine points and/or line intersections.

4. Device according to one of the preceding claims, in which the inertial sensor (41) is firmly fixed to the screen (13, 23, 33, 43) and supplies the inertial information to the processing unit (42); the inertial information being representative of an inertial orientation of said screen (13, 23, 33, 43) .

5. Device according to one of the preceding claims, in which the inertial sensor (41) comprises one or more of the types of sensors selected from the following list:
- one or more accelerometers,
- gyroscope,
- inertial reference system,
- magnetic field detector.

6. Device according to one of claims 4 or 5, in which the processing unit (42) is configured to produce an image containing the inertial matrix oriented and displaced on the screen (13, 23, 33, 43) as a function of the inertial information received from the sensor (41).

7. Device according to the preceding claim, in which the processing unit (42) is configured to:
- produce a virtual matrix (500) around the user having a position fixed in space,
- position the screen with respect to the virtual matrix as a function of the inertial information, and
- extract the image representative of the inertial matrix to be displayed on the screen as a function of the positioning of the screen.

8. Device according to one of the preceding claims, also containing a fastening means (31) for a vehicle seat and at least one linking arm (32) linking the at least one screen (33) to the fastening means (31) such that the screen (33) is placed at the level of one side of the head of a user of the seat, so that the screen (33) is placed in the peripheral visual field of said user.

9. Pair of glasses (10) comprising two sidearms (12) intended to cooperate with the ears of a user, **characterized in that** it contains at least one device according to one of claims 1 to 7, at least one screen (13) being fastened on one of the sidearms (12) such that the screen is placed laterally in the peripheral visual field of the user.

10. Virtual reality mask (20) comprising a housing (21) intended to be placed in the visual field of a user and containing at least one main screen (22) placed in the central visual field of a user, **characterized in that** it contains a device according to one of claims 2 to 7 and in which the two screens (23) are placed laterally inside the housing such that they are placed in the peripheral visual field of a right eye and a left eye of the user, respectively.
